# EUROPEAN PATENT SPECIFICATION

(11) **EP 3 624 832 B1**
(45) Date of publication and mention of the grant of the patent: **20.08.2025**
(21) Application number: 18724555.0
(22) Date of filing: 15.05.2018
(51) Int. Cl.: A61K 38/46, A61K 38/49, A61P 9/10

(54) **METHODS AND PHARMACEUTICAL COMPOSITIONS FOR THE TREATMENT OF ACUTE ISCHEMIC STROKE**
VERFAHREN UND PHARMAZEUTISCHE ZUSAMMENSETZUNGEN ZUR BEHANDLUNG VON AKUTEM ISCHÄMISCHEM SCHLAGANFALL
PROCÉDÉS ET COMPOSITIONS PHARMACEUTIQUES POUR LE TRAITEMENT D'UN ACCIDENT VASCULAIRE CÉRÉBRAL ISCHÉMIQUE AIGU

(30) Priority: 16.05.2017 EP 17305556; 01.02.2018 EP 18305106
(43) Date of publication of application: 25.03.2020
(73) Proprietor: Institut National de la Santé et de la Recherche Médicale, 75013 Paris (FR); Université Paris Cité, 75006 Paris (FR); Université Paris XIII Paris-Nord, 93430 Villetaneuse (FR); Assistance Publique-Hôpitaux de Paris (APHP), 75004 Paris (FR); Fondation Ophthalmologique Adolphe De Rothschild, 75019 Paris (FR)
(72) Inventor: DESILLES, Jean-Philippe, 75018 Paris (FR); OLLIVIER, V ronique, 75018 Paris (FR); HO-TIN-NOE, Benoit, 75018 Paris (FR); MAZIGHI, Mikhael, 75018 Paris (FR); LOYAU, St phane, 75018 Paris (FR); MICHEL, Jean-Baptiste, 75018 Paris (FR); PIOTIN, Michel, 75019 Paris (FR); DUCROUX, C lina, 75004 Paris (FR); DI MEGLIO, Lucas, 75019 Paris (FR); BLANC, Rapha l, 75019 Paris (FR); BOISSEAU, William, 75004 Paris (FR)
(74) Representative: Inserm Transfert
(86) International application number: PCT/EP2018/062588
(87) International publication number: WO 2018/210860

(56) References cited:
- WO-A1-2012/085933
- WO-A2-2007/084544
- DE MEYER SIMON F ET AL: "Extracellular Chromatin Is an Important Mediator of Ischemic Stroke in Mice", ARTERIOSCLEROSIS THROMBOSIS AND VASCULAR BIOLOGY, vol. 32, no. 8, August 2012 (2012-08-01), pages 1884, XP009501573

## Description

### FIELD:

The disclosure relates to a pharmaceutical composition for the treatment of acute ischemic stroke.

### BACKGROUND:

Acute ischemic stroke (AIS) is the sudden blockage of adequate blood flow to a section of the brain, usually caused by a thrombus or other emboli lodging or forming in one of the arteries supplying the brain. If this blockage is not quickly removed, the ischemia may lead to permanent neurologic deficit or death. Intravenous (IV) administration of recombinant tissue-type plasminogen activator (t-PA; Alteplase, Actilyse^{®}, Boehringer Ingelheim) is the only drug therapy validated in AIS. This treatment has a low efficiency in terms of early recanalization. A pharmaceutical composition comprising a derivative of t-PA for the treatment of acute ischemic stroke is known in the prior art (WO 2012/085933 A1). Since 2015, this treatment is associated, in case of proximal intracranial arterial occlusion, to the realization of endovascular treatment (EVT) allowing an increase in early recanalization rate and a significant improvement of functional outcome at 3 months. The timeframe for AIS treatment is within 4,5 hours for IV t-PA infusion and 6 hours for EVT. Even within this time period, there is strong evidence that the shorter the time period between onset of symptoms and treatment, the better the results. EVT of AIS consists in the mechanical removal of the thrombus with thrombectomy devices such as stent-retrievers or direct aspiration catheters.

The main objective of AIS management is to obtain an early recanalization of the occluded artery as soon as possible. This early recanalization is indeed a major predictive factor of neurological clinical outcome. International guidelines for AIS treatment recommend that IV thrombolysis with t-PA to be performed within 4h30 following the onset of symptoms associated with EVT in cases of proximal occlusion of the anterior circulation in 6 hours. However, IV thrombolysis is not very effective in terms of recanalization in the setting of proximal occlusion. Indeed, rates of 5% in case of occlusion of the internal carotid artery and 20% in occlusion of the proximal segment of the middle cerebral artery have been recently reported. The efficacy of IV t-PA infusion is limited to 10% due to: dilution of the potent compound in the whole blood, inhibition of t-PA by circulating inhibitors such as PAI-1 during its plasma traffic, low initial level of t-PA binding to the thrombus and an increased risk of delayed hemorrhagic transformation. Furthermore, although thrombolytic effects of t-PA are beneficial, its toxicity, at the required high dose ranges that are presently used, is problematic. EVT is associated with high rates of recanalization but is a hyper-specialized, expensive and therefore difficult to access in emergency. An easily administrable drug treatment to increase the recanalization rate associated with IV thrombolysis would represent a major advance in the AIS management.

Neutrophil extracellular traps (NETs) have been recently identified as major triggers and structural factors of various forms of thrombi. NETs are extracellular webs primarily composed of DNA from neutrophils. Recently, a study designated extracellular DNA threads from NETs as a potential therapeutic target for increasing the efficacy of t-PA-induced thrombolysis in acute coronary syndrome (Mangold, A., Alias, S., Scherz, T., Hofbauer, T., Jakowitsch, J., Panzenböck, A. & Mascherbauer, J. (2015). Coronary Neutrophil Extracellular Trap Burden and Deoxyribonuclease Activity in ST-Elevation Acute Coronary Syndrome Are Predictors of ST-Segment Resolution and Infarct Size. Circulation research, 116(7), 1182-1192). Recently, in a mouse model of cerebral ischemia-reperfusion, DNAse 1 alone was found to significantly reduce the infarct volume compared with vehicle (De Meyer, S. F., Suidan, G. L., Fuchs, T. A., Monestier, M., & Wagner, D. D. (2012). Moreover, study demonstrated that a treatment with recombinant human DNAse I significantly improved stroke outcome (De Meyer Simon F et al, arteriosclerosis thrombosis and vascular biology, 2012). Extracellular chromatin is an important mediator of ischemic stroke in mice. Arteriosclerosis, thrombosis, and vascular biology, 32(8), 1884-1891). Thus the study disclosed that DNAse 1 would be suitable for improving the downstream effects induced by the ischemia but failed to disclose that the enzyme would provide a favourable impact on proximal arterial recanalization rate.

### SUMMARY OF THE INVENTION:

The present invention is defined by the claims. In particular, the invention relates to :
- a combination comprising tissue-type plasminogen activator (t-PA) and DNAse for use in a method of treatment of an acute ischemic stroke (AIS) in a patient in need thereof,
- a combination of t-PA and DNAse for use in a method of treatment of an acute ischemic stroke (AIS) in a patient in need thereof, wherein administration of the combination results in enhanced therapeutic efficacy relative to the administration of t-PA alone and,
- a combination of t-PA and DNAse for use in a method of achieving recanalization of occluded intracranial arteries in a patient suffering from an AIS.

The following detailed description, figures and example do not fall under the scope of the present invention and are present for understanding and illustration purposes only. Furthermore, any reference in the description to methods of treatment refer to the compounds, pharmaceutical compositions and medicaments of the present invention for use in a method of treatment of the human body by therapy.

### DETAILED DESCRIPTION OF THE :

Neutrophil Extracellular Traps (NETs) are DNA extracellular networks decorated with histones and granular proteins produced by activated neutrophils. NETs have been identified as major triggers and structural factors of thrombosis. The aim of the inventors was to assess the presence of NETs in thrombi retrieved during endovascular therapy in patients with acute ischemic stroke (AIS) and their impact on t-PA-induced thrombolysis. Therefore they analysed thrombi from 108 AIS patients treated with endovascular therapy. Thrombi were characterized by hematoxylin/eosin staining, immunostaining, and ex vivo enzymatic assay. Additionally, the inventors assessed ex vivo the impact of deoxyribonuclease 1 (DNAse 1) on thrombolysis of AIS thrombi. Histological analysis revealed that NETs contributed to the composition of all AIS thrombi especially in their outer layers. Quantitative measurement of thrombus NETs content was not associated with clinical outcome or AIS pathogenesis but correlated significantly with endovascular therapy procedure length and device number of passes. Ex vivo, recombinant DNAse 1 accelerated t-PA-induced thrombolysis, whereas DNAse 1 alone was ineffective. This study suggests that thrombus NETs content may be responsible for reperfusion resistance, including mechanical or pharmacological approaches with intravenous t-PA, irrespectively of their etiology. These results also suggest that a co-therapy associating t-PA and DNAse 1 may have synergistic action that potentialize t-PA efficacy.

The first object of the disclosure relates to a method of treating an acute ischemic stroke (AIS) in a patient in need thereof comprising administering to the patient t-PA and DNAse. In particular, the method of the disclosure comprises administering to the patient a therapeutically effective combination of t-PA and DNAse.

As used herein, the terms "therapeutically effective amount" or "therapeutically effective combination" or "pharmaceutically effective amount" refer to the amount or dose of t-PA, to the amount or dose of DNAse, or to the amount or dose of both, in a combination according to the disclosure, that is aimed at, without causing significant negative or adverse side effects to the subject, (1) delaying or preventing the onset of AIS; (2) reducing the severity or incidence of AIS; (3) slowing down or stopping the progression, aggravation, or deterioration of one or more symptoms of AIS; (4) bringing about ameliorations of the symptoms of AIS; or (5) curing AIS. A therapeutically effective amount or combination may be administered prior to the onset of AIS. Alternatively, or additionally, a therapeutically effective amount or combination may be administered after initiation of AIS.

In particular, the administration of the combination of t-PA and DNAse results in enhanced therapeutic efficacy relative to the administration of t-PA alone.

A further object of the disclosure relates to a method for enhancing the potency of t-PA administered to a patient suffering from an AIS as part of a treatment regimen, the method comprising administering to the patient a pharmaceutically effective amount of t-PA in combination with DNAse.

A further object of the disclosure relates to a method for enhancing the potency of t-PA administered to a patient suffering from an AIS as part of a treatment regimen, the method comprising administering DNAse to the patient treated with t-PA. In particular, a pharmaceutically effective amount of DNAse is administered to the patient. In particular, the method of the disclosure allows decreasing the dose of t-PA to be administered to the patient.

A further object of the present disclosure relates to a method of achieving recanalization of occluded intracranial arteries in a patient suffering from an AIS comprising administering to the patient t-PA and DNAse. In particular, the method of the disclosure comprises administering to the patient a therapeutically effective combination of t-PA and DNAse.

The present disclosure further relates to a combination comprising or consisting of or consisting essentially of t-PA and DNAse for use in the treatment of AIS in a patient in need thereof

As used herein, the term "consisting essentially of", in reference to a combination, means that t-PA and DNase are the only therapeutic compounds or compounds with a biologic activity in the combination of the disclosure.

Another object of the present disclosure is a pharmaceutical combination comprising or consisting of or consisting essentially of t-PA and DNAse, and at least one pharmaceutically acceptable carrier, for use in the treatment of AIS in a patient in need thereof.

Another object of the present disclosure is a kit-of-parts comprising a first part comprising t-PA and a second part comprising DNAse for use in the treatment of AIS in a patient in need thereof.

Another object of the present disclosure is a kit-of-parts comprising a first part comprising a pharmaceutical composition comprising t-PA and at least one pharmaceutically acceptable carrier, and a second part comprising a pharmaceutical composition comprising DNAse and at least one pharmaceutically acceptable carrier, for use in the treatment of AIS in a patient in need thereof.

Another object of the disclosure is a medicament comprising a combination of t-PA and DNAse as described hereinabove, or a pharmaceutical combination as described hereinabove, or a kit-of parts as described hereinabove, for use in the treatment of AIS in a patient in need thereof.

As used herein, the term "acute ischemic stroke" or 'AIS" refers to those patients having or at risk for "definite acute ischemic cerebrovascular syndrome (AICS)" as defined by the diagnostic criteria of Kidwell et al. "Acute Ischemic Cerebrovascular Syndrome: Diagnostic Criteria," Stroke, 2003, 34, pp. 2995-2998. Accordingly, acute ischemic stroke refers to an acute onset of neurologic dysfunction of any severity consistent with focal brain ischemia.

In particular, the patient is diagnosed with AIS.

In particular, the patient is at risk of developing AIS. Examples of risk factors for the development of AIS comprise atrial fibrillation or other cardio-embolic diseases, atheromatous plaque or stenosis developed in aortic arch, cervical arteries or intracranial arteries, spontaneous cervical dissection, genetic predisposition, familial history of AIS and the like.

In particular, the patient is a human. In particular, the patient is a male. In particular, the patient is a female.

In particular, the patient was previously treated by EVT, or is planned to be treated with EVT. Therefore, in particular, the method of the disclosure comprises administering t-PA and DNAse to a patient, and carrying out an EVT in said patient.

As used herein, the term "treatment" or "treat" refer to both prophylactic or preventive treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (i.e. administering a drug at a regular intervals, e.g., weekly, monthly, yearly, etc.) or intermittent therapy (i.e. interrupted treatment, intermittent treatment, treatment at relapse, or treatment upon achievement of a particular predetermined criteria [e.g., disease manifestation, etc.]).

As used herein, the term "t-PA" has its general meaning in the art and refers to tissue-type plasminogen activator. The term includes native t-PA and recombinant t-PA, as well as modified forms of t-PA that retain the enzymatic or fibrinolytic activities of native t-PA. The enzymatic activity of t-PA can be measured by assessing the ability of the molecule to convert plasminogen to plasmin. The fibrinolytic activity of t-PA may be determined by any in vitro clot lysis activity known in the art. Recombinant t-PA has been described extensively in the prior art and is known to the person of skill. t-PA is commercially available as alteplase (Activase^{®} or Actilyse^{®}). Modified forms of t-PA ("modified t-PA") have been characterized and are known to those skilled in the art. Modified t-PAs comprise variants having deleted or substituted amino acids or domains, variants conjugated to or fused with other molecules, and variants having chemical modifications, such as modified glycosylation. Several modified t-PAs have been described in PCT Publication No. WO93/24635; EP 352,119; EP382174. In particular, the modified form of t-PA is Tenecteplase. As used herein, the term "tenecteplase," also known as TNK-t-PA or TNKASE^{™} brand of tissue-plasminogen activator variant, refers to a t-PA variant designated T103N, N117Q, K296A, H297A, R298A, R299A t-PA available from Genentech, Inc. (South San Francisco Calif.) wherein Thr103 of wild-type t-PA is changed to Asn (T103N), Asn 117 of wild-type t-PA is changed to Gln (N117Q), and Lys-His-Arg-Arg 296-299 of wild-type t-PA is changed to Ala-Ala-Ala-Ala (KHRR296-299AAAA). Tenecteplase is a genetically engineered variant of human t-PA cloned and expressed in Chinese hamster ovary cells (see Keyt et al., Proc. Natl. Acad. Sci USA, 91: 3670-3674 (1994) and Verstraete, Am. J. Med, 109: 52-58 (2000) for an overview of third-generation thrombolytic drugs in general). Tenecteplase was engineered to have increased fibrin specificity and an increased half-life compared to alteplase.

In particular, the t-PA is a mutated t-PA as described in. In particular, said mutated t-PA comprises the sequence SEQ ID NO: 1 (corresponding to human wt t-PA mature form) or SEQ ID NO: 2 (corresponding to human wt t-PA first chain of tc-t-PA), preferably consisting of SEQ ID NO: 1 or of the association of SEQ ID NO: 2 and SEQ ID NO: 3 (corresponding to human wt t-PA second chain of tc-t-PA), or variant thereof having at least 80%, 85%, 90%, 95% or more identity, wherein said sequence comprises a mutation consisting of the replacement of any amino acid of the Lysine Binding Site of SEQ ID NO: 1 or SEQ ID NO: 2 by a hydrophilic amino acid chosen from arginine, aspartic acid, glutamic acid, lysine, asparagine, glutamine, serine, threonine, tyrosine and histidine, preferably by arginine, and/or a mutation consisting of the replacement of arginine in position 275 of SEQ ID NO: 1 or SEQ ID NO: 2 by serine.

In particular, said mutated t-PA comprises the sequence SEQ ID NO: 1 (corresponding to human wt t-PA mature form) or SEQ ID NO: 2 (corresponding to human wt t-PA first chain of two chain t-PA), preferably consisting of SEQ ID NO: 1 or of the association of SEQ ID NO: 2 and SEQ ID NO: 3 (corresponding to human wt t-PA second chain of two chain t-PA), or variant thereof having at least 80%, 85%, 90%, 95% or more identity, wherein said sequence comprises a mutation consisting of the replacement of tryptophan in position 253 of SEQ ID NO: 1 or SEQ ID NO: 2 by a hydrophilic amino acid chosen from arginine, aspartic acid, glutamic acid, lysine, asparagine, glutamine, serine, threonine, tyrosine and histidine, preferably by arginine, and/or a mutation consisting of the replacement of arginine in position 275 of SEQ ID NO: 1 or SEQ ID NO: 2 by serine.

In particular, said mutated t-PA further comprises at least one of the following mutations:
- the replacement of proline in position 125 of SEQ ID NO: 1 or SEQ ID NO: 2 by arginine,
- the deletion of the Finger domain in the N-terminus and/or the deletion of the EGF-like domain, in SEQ ID NO: 1 or SEQ ID NO: 2, and/or the replacement of asparagine in position 117 of SEQ ID NO: 1 or SEQ ID NO: 2 by glutamine,
- the replacement of threonine in position 103 of SEQ ID NO: 1 or SEQ ID NO: 2 by asparagine, and/or the replacement of asparagine in position 117 of SEQ ID NO: 1 or SEQ ID NO: 2 by glutamine, and/or the replacement of lysine-histidine-arginine-arginine (KHRR) in positions 296 to 299 of SEQ ID NO: 1 by alanine-alanine-alanine-alanine (AAAA),

- the replacement of cysteine in position 84 of SEQ ID NO: 1 or SEQ ID NO: 2 by serine,
- the replacement of arginine in position 275 of SEQ ID NO: 1 or SEQ ID NO: 2 by glutamic acid or glycine, and/or the deletion of the Kringle 1 domain in SEQ ID NO: 1 or SEQ ID NO: 2.

As used herein, the term "identity" or "identical", when used in a relationship between the sequences of two or more amino acid sequences, refers to the degree of sequence relatedness between amino acid sequences, as determined by the number of matches between strings of two or more amino acid residues. "Identity" measures the percent of identical matches between the smaller of two or more sequences with gap alignments (if any) addressed by a particular mathematical model or computer program (*i.e.*, "algorithms"). Identity of related amino acid sequences can be readily calculated by known methods. Such methods comprise those described in Arthur M. Lesk, Computational Molecular Biology: Sources and Methods for Sequence Analysis (New-York: Oxford University Press, 1988); Douglas W. Smith, Biocomputing: Informatics and Genome Projects (New-York: Academic Press, 1993); Hugh G. Griffin and Annette M. Griffin, Computer Analysis of Sequence Data, Part 1 (New Jersey: Humana Press, 1994); Gunnar von Heinje, Sequence Analysis in Molecular Biology: Treasure Trove or Trivial Pursuit (Academic Press, 1987); Michael Gribskov and John Devereux, Sequence Analysis Primer (New York: M. Stockton Press, 1991); and Carillo et al., 1988. SIAM J. Appl. Math. 48(5):1073-1082. Preferred methods for determining identity are designed to give the largest match between the sequences tested. Methods of determining identity are described in publicly available computer programs. Preferred computer program methods for determining identity between two sequences include the GCG program package, including GAP (Devereux et al., 1984. Nucl. Acid. Res. 12(1 Pt 1):387-395; Genetics Computer Group, University of Wisconsin Biotechnology Center, Madison, WI), BLASTP, BLASTN, TBLASTN and FASTA (Altschul et al., 1990. J. Mol. Biol. 215(3):403-410). The BLASTX program is publicly available from the National Center for Biotechnology Information (NCBI) and other sources (BLAST Manual, Altschul et al. NCB/NLM/NIH Bethesda, Md. 20894; Altschul et al., 1990. J. Mol. Biol. 215(3):403-410). The well-known Smith Waterman algorithm may also be used to determine identity.

As used herein, the term "DNAse" has its general meaning in the art and refers to and includes all enzymes having a phosphodiesterase activity and the ability to hydrolyse DNA. Any suitable DNAse may be used in the present disclosure. The DNAse will most preferably be a DNAse 1 (EC 3.1.21.1). It may, however, in particular be a DNAse II (EC 3.1.21.1). DNAses occur in a number of species and any DNAse capable of cleaving DNA may be used in the disclosure. In particular, the DNAse is recombinant DNAse. The DNAse may be from an animal source such as of bovine or porcine origin. It may be of plant, fungal, or microbial origin. However, typically and most preferably the DNAse is of human origin and is preferably a recombinant human DNAse. Commercially available DNAse preparations such as Dornase^{™} and Pulmozyme^{™} may be used in the disclosure.

As used herein, the term "combination" is intended to refer to all forms of administration that provide a first drug together with a further (second, third...) drug.

The drugs may be administered simultaneously, separately or sequentially and in any order. In particular, t-PA is administered prior to the administration of DNAse. In particular, DNAse in administered prior to t-PA administration. In particular, t-PA and DNAse are administered simultaneously.

Drugs administered in combination have biological activity in the subject to which the drugs are delivered. Within the context of the disclosure, a combination thus comprises at least two different drugs, and wherein one drug is at least t-PA and wherein the other drug is DNAse. As used herein, the expression "enhancing the potency of t-PA" refers to the ability of the recombinant DNAse to increase fibrinolysis induced by t-PA. In particular, DNAse is suitable for increasing the recanalization rate provided by t-PA. The combination of the 2 drugs thus results in the acceleration of thrombus fibrinolysis.

As used herein, the term "therapeutically effective combination" as used herein refers to an amount or dose of t-PA together with the amount or dose of DNAse that is sufficient to treat the acute ischemic stroke and in particular for achieving recanalization of occluded arteries. The amount of the t-PA or DNAse in a given therapeutically effective combination may be different for different individuals and different diseases, and will be dependent upon the one or more additional agents or treatments included in the combination. The "therapeutically effective amount" is determined using procedures routinely employed by those of skill in the art such that an "improved therapeutic outcome" results. It will be understood, however, that the total daily usage of the compounds and compositions of the present disclosure will be decided by the attending physician within the scope of sound medical judgment. The specific therapeutically effective dose level for any particular subject will depend upon a variety of factors including the disorder being treated and the severity of the disorder; activity of the specific compound employed; the specific composition employed, the age, body weight, general health, sex and diet of the subject; the time of administration, route of administration, and rate of excretion of the specific compound employed; the duration of the treatment; drugs used in combination or coincidential with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Typically, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day. In particular, a single dose of the t-PA, of the DNAse or of the combination of the present disclosure is administered (or is to be administered) to the subject, preferably during the acute phase of an AIS. In particular, multiple doses of the t-PA, of the DNAse or of the combination of the present disclosure are administered (or are to be administered) to the subject. Therefore, in particular, the combination of the disclosure is administered as divided doses. In particular, the dose of t-PA administered (or to be administered) to the subject is comprised between about 0.01 mg/kg and about 5 mg/kg. In particular, the dose of t-PA administered to the subject is comprised between about 0.1 mg/kg and about 1 mg/kg. In particular, the dose of Alteplase (t-PA of the present disclosure) administered to the subject is equal to about 0.9 mg/kg. In particular, the dose of Tenecteplase (t-PA of the present disclosure) administered to the subject is comprised between about 0.1 mg/kg and about 0.4 mg/kg. In particular, the dose of DNAse of the present disclosure administered to the subject is comprised between about 10 µg/kg and about 500 µg/kg, preferably between about 50 µg/kg and about 250 µg/kg. In particular, the dose of rhDNAse 1 (DNAse of the present disclosure) administered to the subject is equal to about 125 µg/kg. In particular, the method of the disclosure thus comprises the administration to the subject of a dose of t-PA ranging from about 0.01 mg/kg to about 5 mg/kg, and the administration to the subject of a dose of DNAse ranging from about 0.1 µg/kg and about 500 µg/kg. As used herein, the term "about", preceding a figure means plus or less 10% of the value of said figure.

Typically, the drug of the present disclosure (*i.e.*, the t-PA or DNAse or combination thereof) is administered to the subject in the form of a pharmaceutical composition which comprises a pharmaceutically acceptable carrier.

As used herein, the term "pharmaceutically acceptable carrier" refers to an excipient that does not produce an adverse, allergic or other untoward reaction when administered to a mammal, preferably a human. It includes any and all solvents, dispersion media, coatings, antibacterial and antifungal agents, isotonic and absorption delaying agents and the like. A pharmaceutically acceptable carrier refers to a non-toxic solid, semi-solid or liquid filler, diluent, encapsulating material or formulation auxiliary of any type. For human administration, preparations should meet sterility, pyrogenicity, general safety and purity standards as required by the regulatory offices such as the FDA or EMA.

Pharmaceutically acceptable carriers that may be used in these compositions comprise ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene- block polymers, polyethylene glycol and wool fat. For use in administration to a subject, the composition will be formulated for administration to the subject.

The compositions of the present disclosure may be administered orally, parenterally, by inhalation spray, topically, rectally, nasally, buccally, vaginally or via an implanted reservoir.

In particular, t-PA and DNAse, the combination or pharmaceutical combination thereof, medicament or kit-of-parts according to the disclosure will be formulated for administration to the subject. t-PA and DNAse, the combination or pharmaceutical combination thereof, or medicament according to the disclosure may be administered orally, parenterally, topically, by inhalation spray, rectally, nasally, buccally, vaginally or via an implanted reservoir.

In particular, t-PA and DNAse are administered to the patient via the same administration route. In particular, t-PA and DNAse are administered to the patient via different administration routes.

In particular, t-PA and DNAse, the combination or pharmaceutical combination thereof, medicament or kit-of-parts according to the disclosure is injected. The used herein includes subcutaneous, intravenous, intramuscular, intra-articular, intra-synovial, intrasternal, intrathecal, intrahepatic, intralesional and intracranial injection or infusion techniques. Sterile injectable forms of the compositions or combinations of this disclosure may be an aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono-or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

The compositions or combinations of this disclosure may be orally administered in any orally acceptable dosage form comprising capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include, e.g., lactose. When aqueous suspensions are required for oral use, the active ingredient is combined with emulsifying and suspending agents. If desired, certain sweetening, flavoring or coloring agents may also be added.

Alternatively, the compositions or combinations of this disclosure may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

The compositions or combinations of this disclosure may also be administered topically, especially when the target of treatment includes areas or organs readily accessible by topical application, including diseases of the eye, the skin, or the lower intestinal tract. Suitable topical formulations are readily prepared for each of these areas or organs. For topical applications, the compositions may be formulated in a suitable ointment containing the active component suspended or dissolved in one or more carriers. Carriers for topical administration of the compounds of this disclosure comprise mineral oil, liquid petrolatum, white petrolatum, propylene glycol, polyoxyethylene, polyoxypropylene compound, emulsifying wax and water. Alternatively, the compositions can be formulated in a suitable lotion or cream containing the active components suspended or dissolved in one or more pharmaceutically acceptable carriers. Suitable carriers comprise mineral oil, sorbitan monostearate, polysorbate 60, cetyl esters wax, cetearyl alcohol, 2-octyldodecanol, benzyl alcohol and water. Topical application for the lower intestinal tract can be effected in a rectal suppository formulation (see above) or in a suitable enema formulation. Patches may also be used.

The compositions of this disclosure may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

The disclosure will be further illustrated by the following figures and examples. However, these examples and figures should not be interpreted in any way as limiting the scope of the present disclosure.

### FIGURES:

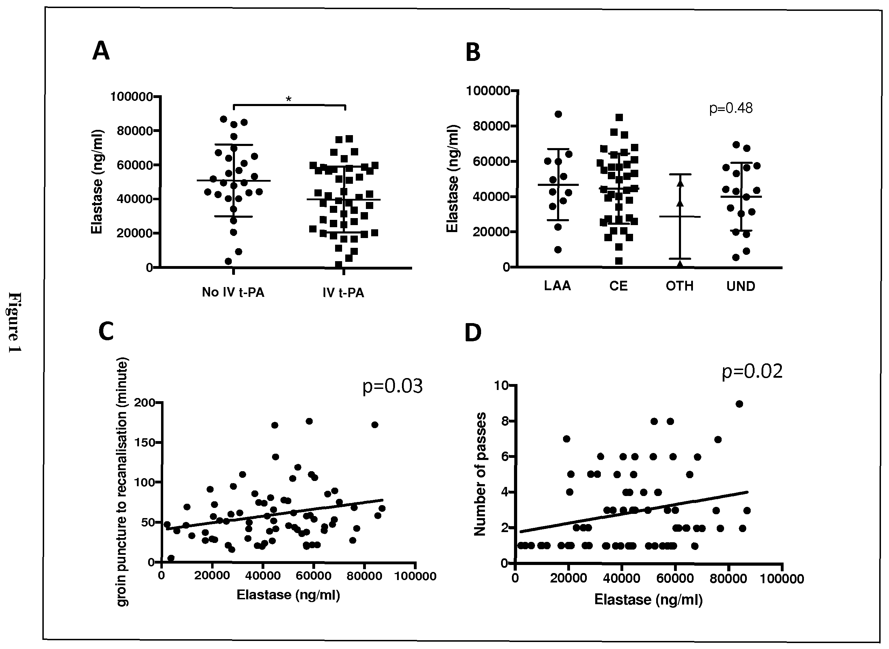

**Figure 1****. Correlation between neutrophil extracellular traps (NETS) burden, acute thrombolytic therapy, and stroke etiology.** To assess the correlation between NETs burden and acute ischemic stroke patients' characteristics, thrombus NETs content was determined by quantifying the release of neutrophil elastase by endonuclease-treated thrombi. (A), NETS burden was slightly but significantly reduced in patients treated with IV t-PA compared with those that did not receive t-PA treatment. (B) There was no association between stroke etiology and NETS burden according to the TOAST classification (LAA : large-artery atherosclerosis ; CE : cardioembolism ; LAC : lacunar ; OTH : other cause ; UND : undetermined cause). (C) and (D) Interestingly, NETs burden was positively correlated with EVT intervention length and the number of device passes.

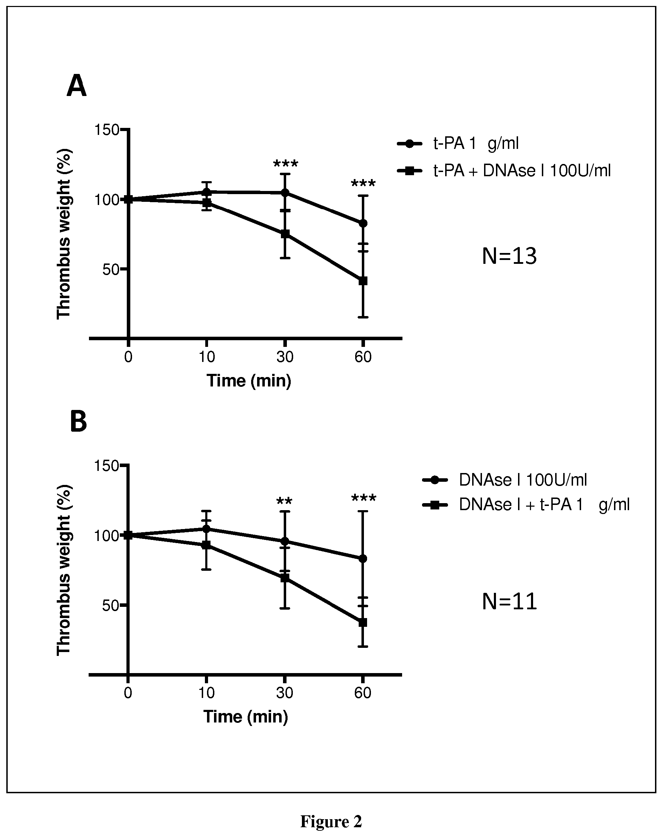

**Figure 2****. Ex vivo stroke clot lysis assay. The thrombolytic efficacy of tPA and DNAse administered alone or in combination was compared in vitro.** For these experiments, thrombectomy-recovered thrombi were incubated in PBS supplemented with tPA (1µg/mL) and/or DNAse (100 U/mL). Thrombus weight was assessed before and at 10, 30 and 60 min following the addition of tPA and DNAse. Results are expressed as percent relative to the initial thrombus weight. Data are presented as medians (interquartile range).

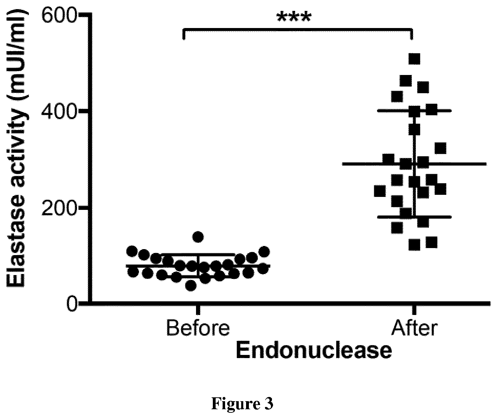

**Figure 3****: Neutrophil extracellular traps are constitutively present in acute ischemic stroke thrombi.** The presence of NETs in AIS thrombi was investigated by measurement of DNA-associated neutrophil elastase activity. The dot plot shows the elastase activity measured in supernatants of thrombi before and after endonuclease treatment (n=23; p<0,0001).

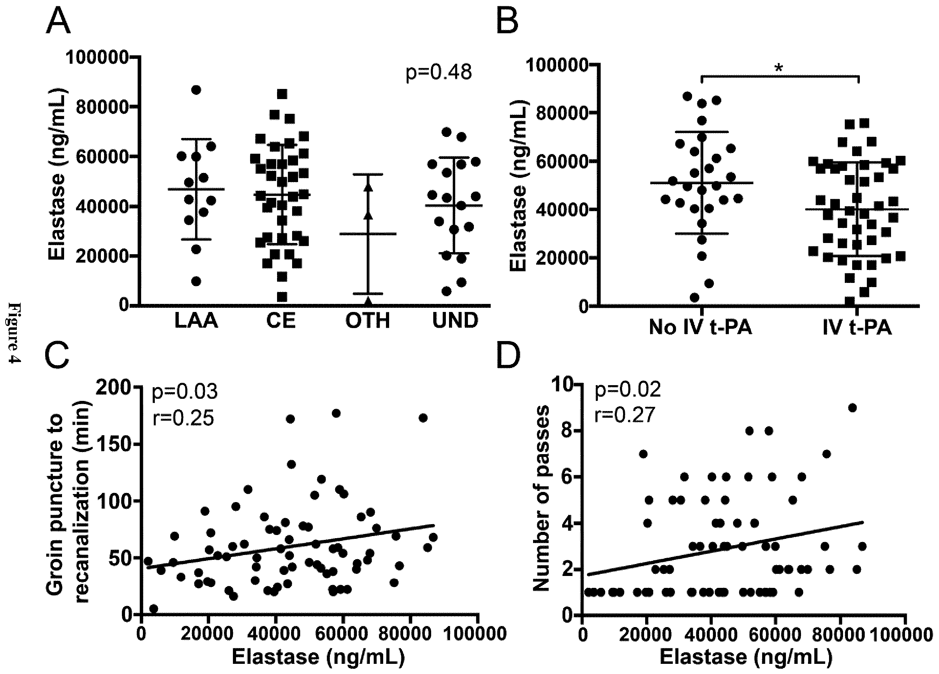

**Figure 4****: Thrombus neutrophil-derived extracellular DNA content is not associated with AIS etiology but with endovascular procedure characteristics.** A-B. Comparison of neutrophil-derived extracellular DNA content (n=72) between thrombi classified according to (A) stroke etiology (LAA: atherosclerosis; CE: cardioembolic; OTH: other cause; UND: undetermined) or (B) to administration or not of IV t-PA treatment prior to endovascular therapy (p=0.03). C-D. Correlation between thrombus neutrophil-derived extracellular DNA content (n=72) and endovascular procedure length (C); and number of device passes achieved (D).

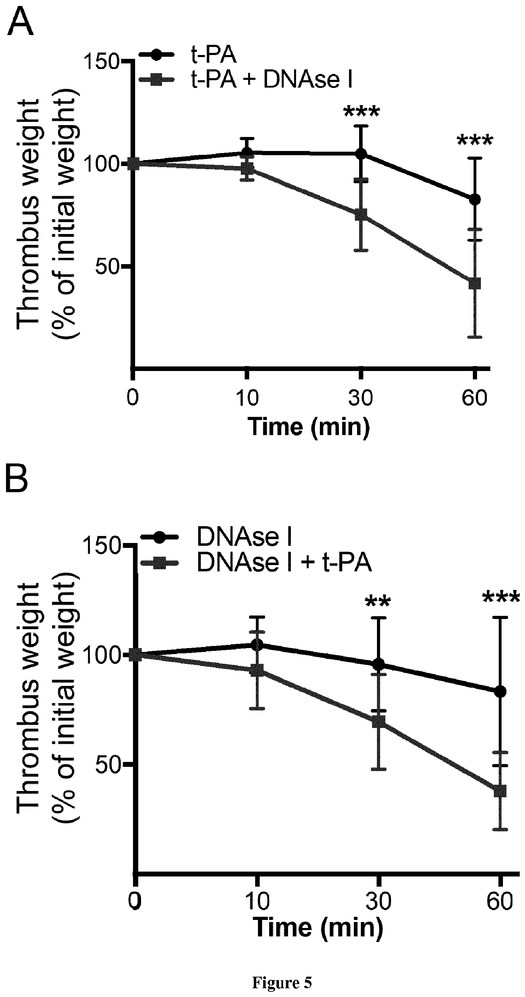

**Figure 5****. DNAse 1 potentiates t-PA-induced thrombolysis ex vivo.** Acute ischemic stroke thrombi recovered by endovascular therapy were incubated with t-PA and/or DNAse I, and their lysis was followed by measurement of thrombus wet-weight evolution over time. Mean baseline weight of thrombi was 14,6 ± 8,4mg. A. Comparison of the thrombolytic effect of t-PA alone or in combination with DNAse I. (n=13; mean [SD]; 10 minutes: t-PA = 105.3% [7.02] versus t-PA+DNAse I = 97.73% [5.62] (p=0.022), 30 minutes: t-PA = 104.8% [13.45] versus t-PA+DNase I = 75.13% [17.39] (p=0.001), 60 minutes: t-PA = 82.71% [20.08] versus t-PA+DNAse I = 41.71% [26.43] (p=0.007). t-PA alone is associated with a slightly but significant thrombus weight reduction at 60 minutes compared to baseline (p=0.003). B. Comparison of the thrombolytic effect of DNAse I alone or in combination with t-PA (n=11, mean [SD]; 10 minutes: DNAse I = 104.6% [12.73] versus DNAse I+ t-PA = 92.91% [17.55], 30 minutes: DNAse I = 95.66% [21.29] versus DNAse + t-PA = 69.39% [21.65], 60 minutes: DNAse I= 83.36% [33.89] versus DNAse I+ t-PA = 37.83% [17.65]). DNAse I alone has no impact on thrombus weight after 60 minutes compared to baseline (p=0.06).

### EXAMPLE 1:

Neutrophil extracellular traps (NETs) have been recently identified as major triggers and structural factors of various forms of thrombosis. NETs are extracellular webs primarily composed of DNA from neutrophils. Recently, a study designated extracellular DNA threads from NETs as a potential therapeutic target for increasing the efficacy of t-PA-induced thrombolysis in acute coronary syndrome. The aim of this study was to assess the impact of NETs burden in thrombi retrieved during endovascular therapy in acute ischemic stroke (AIS) patients on t-PA induced thrombolysis, clinical outcome and AIS etiologies.

We analyzed thrombi from 150 patients with AIS treated with endovascular therapy. Thrombi were characterized by HE staining, immunostaining and enzymatic assays. Histologic analysis revealed that NETs contributed to the scaffold of the majority of thrombi especially in their superficial layers. *In vitro* endonuclease treatment of thrombi revealed an important release in the supernatant of free DNA (p<0.001) and neutrophil elastase (p<0.001) reflecting a quantitative thrombus NETs burden. Thrombus NETs burden was not correlated with clinical outcome or AIS etiology. Recombinant deoxyribonuclease 1 (DNAse 1) accelerated the t-PA-induced thrombolysis in *in vitro* lysis assays (p=0.02) whereas t-PA or DNAse 1 alone was both ineffective to induce a significant thrombolysis of these thrombi.

This study suggests that NETs encapsulated structure of AIS thrombi could participate to the t-PA induced thrombolysis resistance independently of their etiology and localization. The efficacy of a strategy involving a co-administration of DNAse 1 in addition to t-PA to accelerate AIS thrombus fibrinolysis could be very interesting in the treatment of AIS.

### EXAMPLE 2:

### Methods:

### Sample selection

Patients treated in our institution by endovascular therapy (EVT) between December 2015 and December 2016 with successful thrombi retrieval were enrolled in this study. The local Ethics Committee approved this research protocol.

### Data collection

Patient demographics, vascular risk factors, imaging findings, vital signs before treatment, severity of AIS and clinical outcomes were prospectively collected using a structured questionnaire (ETIS registry, Endovascular Treatment in Ischemic Stroke). Stroke etiology was classified using the TOAST classification.

### Endovascular therapy procedure

The EVT procedure was chosen at the interventionalist's discretion, using a stent-retriever or a direct aspiration first path technique in the first instance. The detailed technical procedure has been published previously. 1

### Acute ischemic stroke thrombi collection and processing

Acute ischemic stroke (AIS) thrombi were collected at the end of EVT. They were either immediately frozen at -20°C and then stored at -80°C, and/or fixed in paraformaldehyde 3,7% and then embedded in paraffin. One hundred and eight patients were included. Thrombi from 74 patients were used for lysis assays (n=24) and/or determination of NETs content as assessed by measurement of NE release after endonuclease treatment. NE release was quantified by measurement of NE antigen (n=72) and/or activity (n=23). Thrombi from 34 patients were fixed in paraformaldehyde and used for immunohistological analysis.

### Histology and immunostaining

After deparaffinization, tissue sections were permeabilized, washed, incubated with primary antibodies to MPO (rabbit anti-human MPO antibody, A0398, Dako), citrulinated histone H4 (rabbit anti-human Histone H4 (citrulline 3) antibody, 07-596, Millipore), followed by incubation with fluorescent secondary antibodies and counterstaining with DAPI (Sigma-Aldrich). Hematoxylin/eosin (H&E) staining was also performed in each AIS thrombus.

### Ex vivo NETs assessment

A commercial NETs assay kit (Cayman chemical) was used. It consists in measuring neutrophil elastase activity in tissue supernatant before and after incubation with endonuclease. The released neutrophil elastase corresponds to extracellular DNA-associated neutrophil elastase. To quantify AIS thrombi NETs content, a human neutrophil elastase ELISA kit (HycultBiotech) was used to measure neutrophil elastase antigen in supernatants of thrombi recovered after endonuclease treatment.

### Ex vivo thrombus lysis assay

The following protocol was adapted from Mangold et al.2 Briefly, frozen AIS thrombi were thawed, split into 2 equal parts, and incubated in PBS in the presence of recombinant t-PA (1µg/mL, Actilyse, Boehringer Ingelheim, Ingelheim am Rhein, Germany) and/or recombinant DNAse 1 (100 IE/ml, Pulmozyme, Roche, Basel, Switzerland) on a thermomixer at 500 rpm and at 37°C. Thrombus weight was measured using an ultraprecision balance before and at 10, 30 and 60 minutes after treatment initiation. Thrombus lysis was expressed as percent relative to baseline thrombus weight.

### Statistical analysis

Data were analysed using a nonparametric analysis of variance (Kruskal-Wallis), followed by the Wilcoxon rank-sum test, for comparison of paired data, or by the Mann-Whitney U-test, for comparison of unpaired data. Results are presented as mean±SD for continuous variables and numbers (percentage) for qualitative variables. For statistical analysis, PrismGraph 4.0 software (GraphPad Software, San Diego, CA) was used. Values of P<0.05 were considered statistically significant.

### Results:

### Patient's characteristics

We included 108 patients between December 2015 and December 2016 in this study. Patient characteristics are listed in Table I. These characteristics are similar to those of recently published EVT trials.

### Neutrophil extracellular traps are constitutively present in acute ischemic stroke thrombi

The morphometric analysis of AIS thrombi after H&E staining revealed the presence of numerous polymorphonuclear cells in all thrombi examined. Strands of extracellular nucleic acid suggesting of NETs were found in all 34 thrombi analyzed in histology, especially in their superficial layers (**data not shown**).

Immunofluorescence detection confirmed that areas containing extracellular DNA co-localized with citrullinated histones and granular neutrophils proteins (MPO), which correspond to NETs (**data not shown**). To confirm the presence of NETs in AIS thrombi, we realized an *ex vivo* NETs assay in 23 thrombi. For this assay, NETs presence was investigated by measuring NE activity released from thrombi after endonuclease treatment. NE activity was increased after endonuclease incubation, thus confirming the presence of NETs in all thrombi (**Figure 3**).

### Neutrophil-derived extracellular DNA content correlates with EVT procedure length and device number of passes

Thrombus neutrophil-derived extracellular DNA content was quantified by measuring NE antigen released from thrombi treated with endonuclease (n=72). There was no significant correlation between NETs content and stroke etiology, 3-month functional outcome or final TICI score (**Figure 4A**). NETs thrombus content from patients previously treated with IV t-PA was significantly reduced compared to thrombi from patients without IV t-PA therapy (**Figure 4B**). Finally, NETs content was positively correlated with endovascular procedure length and device number of passes (**Figures 4C-D**).

### Targeting NETs with DNAse 1 accelerated ex vivo t-PA-induced thrombolysis

To test if NETs targeting with DNAse 1 could enhance thrombolysis, we performed an *ex vivo* lysis assay in 24 AIS thrombi. In a first experiment, we compared t-PA alone versus t-PA + DNAse 1 (n=13). The addition of DNAse 1 to t-PA significantly accelerated *ex vivo* thrombolysis. In a second experiment, we compared DNAse 1 alone versus t-PA + DNAse 1 (n=11). DNAse 1 alone was ineffective to induce a significant thrombolysis (**Figure 5A-B**).

### Discussion:

Our study shows that: (1) all AIS thrombi contain NETs irrespectively of the stroke etiology, (2) NETs are predominantly located in the outer layer of AIS thrombi, (3) NETs content is associated with endovascular procedure length and device number of passes, (4) t-PA and DNAse 1 co-administration accelerates *ex vivo* thrombolysis compared to t-PA or DNAse 1 alone.

Our findings are in line with a recent report showing that NETs are important constituents of AIS thrombi.⁶ The latter study found a significant higher amount of NETs in AIS thrombi from cardiac origin compared to non-cardiac thrombi. In the present study, we do not find a correlation between NETs and stroke etiology but, maybe more importantly, show that NETs contribute to the scaffold of thrombi irrespectively of their origin. This particular architecture may participate in t-PA resistance, as suggested by a dramatic increase in *ex vivo* t-PA-induced thrombolysis in the presence of DNAse 1. Our results support recent evidence indicating that DNAse 1 could help to potentiate t-PA-induced lysis of human coronary and AIS thrombi. Notably, we show here that treatment with DNAse 1 alone has no thrombolytic effect *ex vivo,* which indicates that both fibrin and neutrophil-derived extracellular DNA matrix have to be targeted to induce successful thrombolysis.

A previous study has shown that extracellular DNA and histones do modify the structure of fibrin, rendering it more resistant to mechanical and enzymatic destruction.⁷ This fact may explain the observed correlation between NETs content and the number of device passes performed to achieve a successful recanalization. In this perspective, NETs may participate in the interaction between the thrombus and the arterial wall or between the thrombus and the EVT device, thus increasing the difficulty for a successful thrombus removal during EVT.

Interestingly, previous experimental studies have already assessed the impact of DNAse 1 infusion in models of ischemia-reperfusion. In a mouse model of cerebral ischemia-reperfusion, DNAse 1 alone was found to significantly reduce the infarct volume compared to vehicle.⁸ In a myocardial ischemia-reperfusion model in rats, DNAse 1 alone was ineffective but DNAse 1 and t-PA co-administration reduced significantly the infarct size.⁹ These discordant results may be explained by the endogenous expression of t-PA in brain capillaries, which would be in favour of an effect of DNAse 1 alone in brain.¹⁰ Therefore, DNAse 1 administration on top on IV t-PA could have a favourable impact on proximal arterial recanalization rate but also in downstream microvascular thrombosis.

Further optimization of thrombolysis might come from evaluation of the endothelial contribution to t-PA resistance. For instance, the protein C pathway plays a crucial role in coagulation and inflammation. In fact, previous studies have shown that elevated soluble endothelial protein C receptor are associated with thrombolysis resistance in AIS patients with large vessel occlusion.¹¹

Finally, these evidence support a "pharmacological cocktail" for the future of AIS treatment including therapies targeting the thrombus embedded in the large vessel, the activated endothelium and the downstream microvascular thrombosis.¹²

### Conclusions:

NETs form a scaffold responsible, at least in part, for thrombus t-PA resistance. Our data support the concept that DNAse 1 infusion could have a synergistic action to improve the efficacy of IV t-PA-induced thrombolysis in AIS.

**TABLE:**

| | |
|---|---|
| Age, mean±SD, y | 69 |
| Male, n(%) | 5 |

| Stroke etiology, n(%) | |
|---|---|
| LAA | 14 |
| CE | 5 |
| OTH | |
| UND | 3 |
| Previous IV t-PA, n(%) | 6 |

| Occlusion site, n(%) | |
|---|---|
| MCA | 6 |
| ICA | 1 |
| Cervical ICA | 1 |
| BA | |
| Admission NIHSS score, | 1 |

| Final TICI score, n(%) | |
|---|---|
| 3 | 5 |
| 2b | 4 |
| 2a | 1 |
| 1 | 1 |

**Table I. Clinical characteristics of acute ischemic stroke patients from which thrombi were collected and used for this study (n=108).** SD: standard deviation; LAA: large artery atherosclerosis; CE: cardioembolic; OTH: other cause; UND: undetermined; IV: intravenous; MCA: middle cerebral artery; ICA: internal carotid artery; BA: basilar artery; NIHSS: National Institute of Health Stroke Scale; TICI: Thrombolysis In Cerebral Infarction.

### REFERENCES:

Throughout this application, various references describe the state of the art to which this disclosure pertains..
1. Seners P, Turc G, Maier B, Mas JL, Oppenheim C, Baron JC. Incidence and predictors of early recanalization after intravenous thrombolysis: A systematic review and meta-analysis. Stroke. 2016;47:2409-2412
2. Goyal M, Menon BK, van Zwam WH, Dippel DW, Mitchell PJ, Demchuk AM, et al. Endovascular thrombectomy after large-vessel ischaemic stroke: A meta-analysis of individual patient data from five randomised trials. Lancet. 2016;387:1723-1731
3. Martinod K, Wagner DD. Thrombosis: Tangled up in nets. Blood. 2014;123 :2768-2776
4. Brinkmann V, Reichard U, Goosmann C, Fauler B, Uhlemann Y, Weiss DS, et al. Neutrophil extracellular traps kill bacteria. Science. 2004;303:1532-1535
5. Mangold A, Alias S, Scherz T, Hofbauer T, Jakowitsch J, Panzenbock A, et al. Coronary neutrophil extracellular trap burden and deoxyribonuclease activity in st-elevation acute coronary syndrome are predictors of st-segment resolution and infarct size. Circ Res. 2015;116:1182-1192
6. Laridan E, Denorme F, Desender L, Francois O, Andersson T, Deckmyn H, et al. Neutrophil extracellular traps in ischemic stroke thrombi. Ann Neurol. 2017
7. Longstaff C, Varju I, Sotonyi P, Szabo L, Krumrey M, Hoell A, et al. Mechanical stability and fibrinolytic resistance of clots containing fibrin, DNA, and histones. J Biol Chem. 2013;288:6946-6956
8. De Meyer SF, Suidan GL, Fuchs TA, Monestier M, Wagner DD. Extracellular chromatin is an important mediator of ischemic stroke in mice. Arterioscler Thromb Vasc Biol. 2012;32:1884-1891
9. Ge L, Zhou X, Ji WJ, Lu RY, Zhang Y, Zhang YD, et al. Neutrophil extracellular traps in ischemia-reperfusion injury-induced myocardial no-reflow: Therapeutic potential of dnase-based reperfusion strategy. Am J Physiol Heart Circ Physiol. 2015;308:H500-509
10. Zlokovic BV, Wang L, Sun N, Haffke S, Verrall S, Seeds NW, et al. Expression of tissue plasminogen activator in cerebral capillaries: Possible fibrinolytic function of the blood-brain barrier. Neurosurgery. 1995;37:955-961
11. Faille D, Labreuche J, Meseguer E, Huisse MG, Ajzenberg N, Mazighi M. Endothelial markers are associated with thrombolysis resistance in acute stroke patients. Eur J Neurol. 2014;21:643-647
12. Amar AP, Griffin JH, Zlokovic BV. Combined neurothrombectomy or thrombolysis with adjunctive delivery of 3k3a-activated protein c in acute ischemic stroke. Front Cell Neurosci. 2015;9:344

## Claims

1. A combination comprising tissue-type plasminogen activator (t-PA) and DNAse for use in the treatment of an acute ischemic stroke (AIS) in a patient in need thereof.

2. A combination of t-PA and DNAse for use in the treatment of an acute ischemic stroke (AIS) in a patient in need thereof, wherein administration of the combination results in enhanced therapeutic efficacy relative to the administration of t-PA alone.

3. A combination of t-PA and DNAse for use in a method of achieving recanalization of occluded intracranial arteries in a patient suffering from an AIS.

4. The combination for use according to any one of claims 1 to 3, wherein the t-PA is recombinant t-PA.

5. The combination for use according to any one of claims 1 to 4, wherein the t-PA is a modified form of native t-PA that retain the enzymatic or fibrinolytic activities of native t-PA.

6. The combination for use according to any one of claims 1 to 5, wherein the t-PA is a modified form of native t-PA wherein Thr103 of wild-type tPA is changed to Asn (T103N), Asn 117 of wild-type tPA is changed to Gln (N117Q), and Lys-His-Arg-Arg 296-299 of wild-type tPA is changed to Ala-Ala-Ala-Ala.

7. The combination for use according to any one of claims 1 to 6, wherein the t-PA is alteplase or tenecteplase.

8. The combination for use according to any one of claims 1 to 7, wherein the DNAse is DNAse 1.

9. The combination for use according to any one of claims 1 to 8, wherein the DNAse is recombinant.

10. The combination for use according to any one of claims 1 to 9, wherein the DNAse is of human origin.

11. The combination for use according to any one of claims 1 to 10, wherein the DNAse is Dornase or Pulmozyme.

## Patentansprüche

1. Kombination, umfassend Gewebeplasminogenaktivator (t-PA) und DNase zur Verwendung bei der Behandlung eines akuten ischämischen Schlaganfalls (AIS) bei einem Patienten, der diese benötigt.

2. Kombination aus t-PA und DNase zur Verwendung bei der Behandlung eines akuten ischämischen Schlaganfalls (AIS) bei einem Patienten, der diese benötigt, wobei Verabreichung der Kombination zu einer erhöhten therapeutischen Wirksamkeit relativ zu der Verabreichung von t-PA allein führt.

3. Kombination aus t-PA und DNase zur Verwendung in einem Verfahren zum Erreichen von Rekanalisierung von verschlossenen intrakraniellen Arterien bei einem Patienten, der an einem AIS leidet.

4. Kombination zur Verwendung nach einem der Ansprüche 1 bis 3, wobei der t-PA ein rekombinanter t-PA ist.

5. Kombination zur Verwendung nach einem der Ansprüche 1 bis 4, wobei der t-PA eine modifizierte Form von nativem t-PA ist, welche die enzymatischen oder fibrinolytischen Aktivitäten von nativem t-PA beibehält.

6. Kombination zur Verwendung nach einem der Ansprüche 1 bis 5, wobei der t-PA eine modifizierte Form von nativem t-PA ist, wobei Thr103 des Wildtyp-tPA in Asn (T103N) geändert ist, Asn 117 des Wildtyp-tPA in Gin (N117Q) geändert ist und Lys-His-Arg-Arg 296-299 des Wildtyp-tPA in Ala-Ala-Ala-Ala geändert ist.

7. Kombination zur Verwendung nach einem der Ansprüche 1 bis 6, wobei der t-PA Alteplase oder Tenecteplase ist.

8. Kombination zur Verwendung nach einem der Ansprüche 1 bis 7, wobei die DNase DNase 1 ist.

9. Kombination zur Verwendung nach einem der Ansprüche 1 bis 8, wobei die DNase rekombinant ist.

10. Kombination zur Verwendung nach einem der Ansprüche 1 bis 9, wobei die DNase menschlichen Ursprungs ist.

11. Kombination zur Verwendung nach einem der Ansprüche 1 bis 10, wobei die DNase Dornase oder Pulmozyme ist.

## Revendications

1. Combinaison comprenant un activateur du plasminogène de type tissulaire (t-PA) et une DNAse pour utilisation dans le traitement d'un accident vasculaire cérébral ischémique aigu (AIS) chez un patient en ayant besoin.

2. Combinaison de t-PA et de DNAse pour utilisation dans le traitement d'un accident vasculaire cérébral ischémique aigu (AIS) chez un patient en ayant besoin, dans laquelle l'administration de la combinaison donne lieu à une efficacité thérapeutique améliorée par rapport à l'administration de t-PA seul.

3. Combinaison de t-PA et de DNAse pour utilisation dans un procédé de réalisation d'une recanalisation d'artères intracrâniennes occluses chez un patient souffrant d'un AIS.

4. Combinaison pour utilisation selon l'une quelconque des revendications 1 à 3, dans laquelle le t-PA est un t-PA recombinant.

5. Combinaison pour utilisation selon l'une quelconque des revendications 1 à 4, dans laquelle le t-PA est une forme modifiée de t-PA natif qui conserve les activités enzymatiques ou fibrinolytiques du t-PA natif.

6. Combinaison pour utilisation selon l'une quelconque des revendications 1 à 5, dans laquelle le t-PA est une forme modifiée du t-PA natif, dans laquelle leu Thr103 du tPA de type sauvage est changé en Asn (T103N), l'Asn 117 du tPA de type sauvage est changé en Gin (N117Q), et le Lys-His-Arg-Arg 296-299 du tPA de type sauvage est changé en Ala-Ala-Ala-Ala.

7. Combinaison pour utilisation selon l'une quelconque des revendications 1 à 6, dans laquelle le t-PA est l'altéplase ou la ténectéplase.

8. Combinaison pour utilisation selon l'une quelconque des revendications 1 à 7, dans laquelle la DNAse est la DNAse 1.

9. Combinaison pour utilisation selon l'une quelconque des revendications 1 à 8, dans laquelle la DNAse est recombinante.

10. Combinaison pour utilisation selon l'une quelconque des revendications 1 à 9, dans laquelle la DNAse est d'origine humaine.

11. Combinaison pour utilisation selon l'une quelconque des revendications 1 à 10, dans laquelle la DNAse est la Dornase ou la Pulmozyme.
